(19) Europäisches Patentamt
European Patent Office
Office européen des brevets

(11) EP 3 156 054 A1

(12) **EUROPEAN PATENT APPLICATION**
published in accordance with Art. 153(4) EPC

(43) Date of publication:
**19.04.2017 Bulletin 2017/16**

(21) Application number: **15806114.3**

(22) Date of filing: **11.06.2015**

(51) Int Cl.:
***A61K 31/437*** (2006.01) ***A61K 51/00*** (2006.01)
***A61P 1/00*** (2006.01) ***A61P 1/04*** (2006.01)
***A61P 1/12*** (2006.01) ***A61P 25/00*** (2006.01)
***A61P 25/22*** (2006.01) ***A61P 25/24*** (2006.01)
***C07D 471/10*** (2006.01)

(86) International application number:
**PCT/JP2015/066922**

(87) International publication number:
**WO 2015/190568 (17.12.2015 Gazette 2015/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **12.06.2014 JP 2014121061**
**09.01.2015 JP 2015002727**
**31.03.2015 JP 2015070536**

(71) Applicant: **ONO Pharmaceutical Co., Ltd.**
**Osaka-shi, Osaka 541-8526 (JP)**

(72) Inventors:
- **KATSUMATA, Seishi**
**Mishima-gun**
**Osaka 618-8585 (JP)**
- **MITSUI, Katsukuni**
**Mishima-gun**
**Osaka 618-8585 (JP)**

(74) Representative: **Grünecker Patent- und Rechtsanwälte**
**PartG mbB**
**Leopoldstraße 4**
**80802 München (DE)**

# (54) MEDICINE FOR PREVENTING AND/OR TREATING STRESS-INDUCED DISEASES

(57) The present invention relates to a medicine for preventing and/or treating stress-induced diseases, said medicine being characterized in that (1S)-2-acetyl-1-(4-chloro-2-methoxyphenyl)-5-fluoro-1,2,3,9-tetrahydrospiro[β-carboline-4,1'-cyclopropane] is administered at such a specific dose that a brain TSPO occupancy required for the development of the pharmacological activity of the medicine can be obtained.

EP 3 156 054 A1

**Description**

TECHNICAL FIELD

The present invention relates to a medicament for preventing and/or treating stress diseases, said medicament being characterized in that (1S)-2-acetyl-1-(4-chloro-2-methoxyphenyl)-5-fluoro-1,2,3,9-tetrahydrospiro[β-carboline-4,1'-cyclopropane] (hereinbelow, it may be sometimes abbreviated as "the compound of the present invention") is administered at such a specific dose that a brain TSPO occupancy requiring for expressing the pharmacological effects of the medicament can be obtained.

BACKGROUND ART

Stress disease, i.e. a disease caused by stress means that by psychosocial or physical stress stimulation (stressor), the distortion (response to stress) in the body subjected to these stimulations occurs and any disorders in systemic various areas develop. Concretely, since stressor influences on the activities of nervous system, endocrine system and immune system to void these functions, these influences lead to organic lesions in brain itself or peripheral organs. In case of being subjected to excess stress, it causes diseases leading to marked reduce of quality of life (so-called, QOL).

Examples of stress disease include central nervous system diseases (e.g. depression, etc.), digestive system diseases (e.g. irritable bowel syndrome (hereinbelow, it may be sometimes abbreviated as "IBS"), gastric ulcer, etc.), cardiovascular system diseases (e.g. essential hypertension, etc.) and the like. For the therapeutic drugs of these stress diseases, antidepressant drug, antianxiety drug and drug for symptomatic treatment against organic lesions of peripheral organs (e.g. antacids, gastric mucoprotective drugs, etc.) have been developed for the purpose of alleviating psychological stressor. However, despite these drugs become effective to some extent, these addictions, side effects or the like frequently develop. At present, the therapy for these diseases not comes to curative therapy.

Drugs based on various pharmacological effects have been investigated as a therapeutic drug for stress diseases. Among the drug target, TSPO (Translocator protein 18kDa) is a protein also referred to as MBR (mitochondrial benzodiazepine receptor), and is attracting attention as a pharmacological target for stress diseases typified by IBS. It is known that TSPO exists in mitochondrial outer membrane of various cells (e.g. macrophage, microglial cell, reactive astroscyte, etc) and is involved in cholesterol transportation and steroid synthesis (refer to Non-Patent Document 1). Neurosteroid is a type of steroid. At the time of stress condition, it has been thought that a change of brain neurosteroid amount collapses a balance between an excitatory signaling system and an inhibitory signaling system, alters activities in nerve system, immune system and endocrine system and, and causes various stress diseases. Therefore, it is thought that adjusting brain neurosteroid balance, by the administration of a drug which has binding and reguratory activity to brain TSPO, is useful for treating stress diseases represented by IBS, which the balance between an excitatory signaling system and an inhibitory signaling system was disrupted by stressor load.

In recent years it is become known that the rate of drug bound to the brain receptors that is pharmacological target, i.e., receptor occupancy is important for expressing pharmacological effects. And now imaging and digitizing of the receptor occupancy became possible by using PET (Positron Emission Tomography) technology. There are plural reports that using PET technology for confirming the efficacy of a drug targeting brain receptors. Examples of these reports include a drug targeting dopamine D2 receptor (refer to Non-Patent Document 2), ORL 1(opioid receptor-like 1) receptor (refer to Non-Patent Document 3), cannabinoid-1 receptor (refer to Non-Patent Document 4), neurokinin (NK) 1 receptor (refer to Non-Patent Document 5) and the like. On the other hand, in the field of PET technology, since we can't use information obtained by other brain receptors or other drugs of same pharmacological target, it is believed that it is very difficult to determine the effective dose of a novel drug for targeting novel brain receptors. Because, in addition to the receptor occupancy capable of expressing the efficacy and optimal radiotracer (also referred to as radioligand and radioactive probe) are different in each receptor and each drug, there are species differences in the relationship between receptor occupancy and blood kinetics, and in internal kinetics of radiotracer (refer to Non-Patent Document 6). For example, Non-Patent Document 5 written that, in order to aprepitant (a NK1 receptor antagonist) expressing the efficacy, it is necessary to occupy 90% or more brain NK1 receptors, and the plasma concentration at that time is about 100ng/mL or higher.

Also, there are many reports about radiotracers which are targeting TSPO (refer to Non-Patent Document 7). Many of these radiotracers only are used as a biomarker for detecting the tissue expression or the expression level of TSPO on TSPO-related disease (e.g. Alzheimer disease, nerve injury, etc) (refer to Non-Patent Document 8 and Patent Document 1).

The the compound of the present invention is known as Example 38 of Patent Document 2 that is a compound having affinity to MBR (TSPO), and useful for preventing and/or treating stress diseases (e.g. IBS, gastric ulcer, Crohn disease, etc.). Also, the present inventors announced the basic experiment results of the the compound of the present

invention at 41st annual meeting of the Society for Neuroscience (12 November 2011), 42st annual meeting of the Society for Neuroscience (17 October 2012), and 7th annual meeting of the Japanese Society for Molecular Imaging (25 May 2012).

Furthermore, the present inventors already announced the human PET studty results and the clinical study results of the the compound of the present invention at 44st annual meeting of the Society for Neuroscience (November 2014), 88th annual meeting of the Japanese Pharmacological Society (March 2015) and Digestive Disease Week 2015 (May 2015).

However, in these prior art documents, there is found no statement or suggestion about that to determine the optimal specific effective dose of the the compound of the present invention for treating stress diseases by examining the relationship between TSPO occupancy and blood kinetics based on human PET study along with the use of the results obtained in various experimental animals.

PRIOR ART DOCUMENTS

PATENT DOCUMENTS

Patent Document 1: International Publication No. 2010/049819 pamphlet
Patent Document 2: International Publication No. 2006/068164 pamphlet

NON-PATENT DOCUMENTS

Non-Patent Document 1: Proceedings of the National Academy of Science of the United States of America, Vol.89, p.3805-3809, 1977
Non-Patent Document 2: Journal of Clinical Pharmacology, Vol.31, No.4, p.497-502, 2011
Non-Patent Document 3: NeuroImage, Vol.68, p.1-10, 2013
Non-Patent Document 4: Proceedings of the National Academy of Science of the United States of America, Vol.104, No.23, p.9800-9805, 2007
Non-Patent Document 5: Biological psychiatry, Vol.55, P.1007-1012, 2004
Non-Patent Document 6: The Journal of the American Society for Experimental NeuroTherapeutics, Vol.2, No.2, p.226-236, 2005
Non-Patent Document 7: European Journal of Nuclear Medicine and Molecular Imaging, Published online: 22 February 2013
Non-Patent Document 8: The Journal of Nuclear Medicine, Vol.52, No.5, p.677-680, 2011

SUMMARY OF THE INVENTION

PROBLEMS TO BE SOLVED BY THE INVENTION

An object of the present invention is to set an optimal dosage regimen of the the compound of the present invention upon use as a prophylactic and/or therapeutic agent for stress diseases.

MEANS TO SOLVE THE PROBLEMS

The present inventors focused on the relationship between the efficacy study in laboratory animals, the TSPO occupancy in ex vivo study and PET studies, and the blood kinetics, and also took species differences into account to solve the above problems, and as a result, found the optimal specific dosage regimen of the the compound of the present invention with excellent efficacy and safety to achieve a therapeutic effect to patients with stress disease, and consequently have completed the present invention.

That is, the present invention relates to:

[1] A medicament for preventing and/or treating stress diseases, said medicament being characterized in that (1S)-2-acetyl-1-(4-chloro-2-methoxyphenyl)-5-fluoro-1,2,3,9-tetrahydrospiro[β-carboline-4,1'-cyclopropane] represented by formula

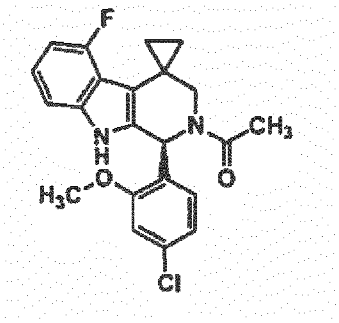

is administered at a dose that makes TSPO occupancy in the patient with stress disease reach about 50 to about 85% per dose,

[2] The medicament according to [1], wherein the dose that makes TSPO occupancy reach about 50 to about 85% is a dose that reaches about 15 to about 150 ng/mL plasma concentration,

[3] The medicament according to [1] or [2], wherein the adminitisration method is an oral administration,

[4] The medicament according to [3], upon oral administration, wherein the dose that makes TSPO occupancy reach about 50 to about 85% is about 5 to about 60 mg,

[5] The medicament according to any one of [1] to [4], wherein the stress disease is irritable bowel syndrome, functional gastrointestinal disorder, depression, or anxiety-related disease,

[6] The medicament according to [5], wherein the stress disease is irritable bowel syndrome,

[7] The medicament according to any one of [1] to [6], wherein the TSPO occupancy was measured using [$^{11}$C] PBR28 as a radiotracer,

[8] A medicament for preventing and/or treating irritable bowel syndrome, said medicament being characterized in that (1S)-2-acetyl-1-(4-chloro-2-methoxyphenyl)-5-fluoro-1,2,3,9-tetrahydrospiro[p-carboline-4,1'-cyclopropane] represented by formula

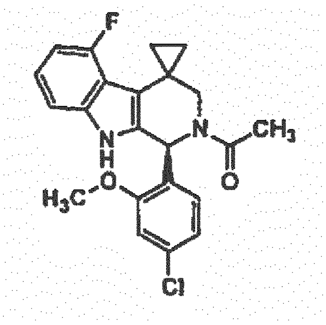

is administered orally at a dose of about 5 to about 60 mg once daily,

[9] A method to set the effective dose of TSPO antagonist for preventing and/or treating stress diseases, said method being characterized in that [$^{11}$C] PBR28 is used as a radiotracer.

[10] A method for preventing and/or treating stress diseases, wherein (1S)-2-acetyl-1-(4-chloro-2-methoxyphenyl)-5-fluoro-1,2,3,9-tetrahydrospiro[β-carboline-4,1'-cyclopropane] represented by formula

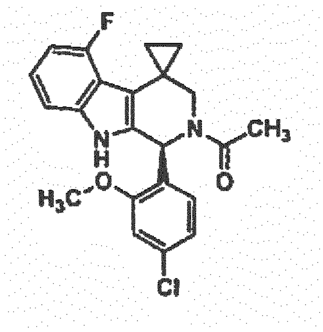

is administered to a patient with stress disease at a dose that makes TSPO occupancy in the patient with stress disease reach about 50 to about 85% per dose,

[11] (1S)-2-acetyl-1-(4-chloro-2-methoxyphenyl)-5-fluoro-1,2,3,9-tetrahydrospiro[β-carboline-4,1'-cyclopropane] represented by formula,

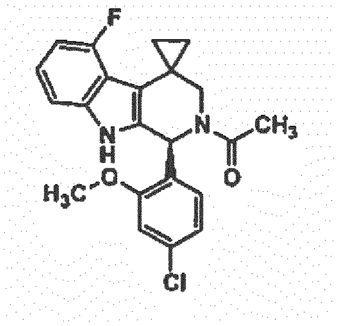

which is administered at a dose that makes TSPO occupancy in the patient with stress disease reach about 50 to about 85% per dose for preventing and/or treating stress diseases,

[12] A medicament for preventing and/or treating stress diseases, said medicament being characterized in that (1S)-2-acetyl-1-(4-chloro-2-methoxyphenyl)-5-fluoro-1,2,3,9-tetrahydrospiro[β-carbotine-4,1'-cyclopropane] represented by formula

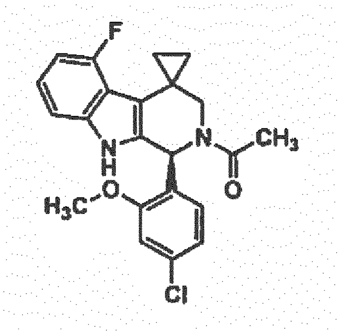

is administered at a dose that makes TSPO occupancy in the patient with stress disease reach about 50 to about 95% per dose,

[13] The medicament according to [12], wherein the dose that makes TSPO occupancy reach about 50 to about 95% is a dose that reaches about 15 to about 250 ng/mL plasma concentration,

[14] The medicament according to claim [12] or [13], wherein the adminitisration method is an oral administration,

[15] The medicament according to [14], upon oral administration, wherein the dose that makes TSPO occupancy reach about 50 to about 95% is about 5 to about 100 mg,

[16] The medicament according to any one of [12] to [15], wherein the stress disease is irritable bowel syndrome, functional gastrointestinal disorder, depression, or anxiety-related disease,

[17] The medicament according to [16], wherein the anxiety-related disease is neurosis, generalized anxiety disorder, social anxiety disorder, panic disorder, hyperactivity disorder, attention deficit, personality disorder, bipolar disorder and autism,

[18] A method for preventing and/or treating stress diseases, comprising (1S)-2-acetyl-1-(4-chloro-2-methoxyphenyl)-5-fluoro-1,2,3,9-tetrahydrospiro[β-carboline-4,1'-cyclopropane] represented by formula

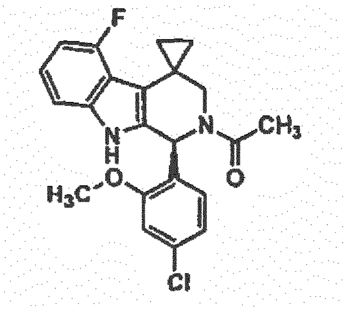

is administered to a patient with stress disease at a dose that makes TSPO occupancy in the patient with stress disease reach about 50 to about 95% per dose, and

[19] (1S)-2-acetyl-1-(4-chloro-2-methoxyphenyl)-5-fluoro-1,2,3,9-tetrahydrospiro[β-carholine-4,1'-cyclopropane] represented by formula,

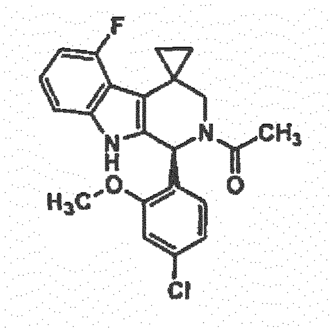

which is administered at a dose that makes TSPO occupancy in the patient with stress disease reach about 50 to about 95% per dose for use in preventing and/or treating stress diseases.

EFFECT OF THE INVENTION

Because the optimal dosage regimen of the the compound of the present invention for treating stress diseases, which was found in the present invention, has excellent efficacy and safety, it can bring a maximal therapeutic effect when using the the compound of the present invention as a medicament for treating stress diseases.

BRIEF EXPLANATION OF THE DRAWINGS

Figure 1: represents antidepressant effect of the the compound of the present invention in olfactory bulb isolated rats.

Figure 2: represents antianxiety effect of the the compound of the present invention in fear conditioning stress rats.

Figure 3: represents antidepressant effect of the the compound of the present invention in CCK-4 induced stress model.

Figure 4: represents relationship between plasma concentration of the the compound of the present invention and brain TSPO occupancy in monkey PET study.

Figure 5: represents relationship between plasma concentration of the the compound of the present invention and brain TSPO occupancy in human PET study.

Figure 6: represents prediction result of relationship between dose and brain TSPO occupancy in steady state when the the compound of the present invention was administered repeatedly once daily to a human based on the Biological example 8. The vertical axis represents TSPO occupancy (Receptor Occupancy (%)). The horizontal axis represents dose of the the compound of the present invention (Dose (mg)).

Figure 7: represents prediction result of relationship between dose and brain TSPO occupancy in steady state when the the compound of the present invention was administered repeatedly once daily to a human based on the Biological example 9. The vertical axis represents TSPO occupancy (Receptor Occupancy (%)). The horizontal axis represents dose of the the compound of the present invention (Dose (mg)).

Figure 8: represents transition of stool consistency score from base line in each treatment group of female diarrhea-type IBS patient. The vertical axis represents amount of change from baseline. The horizontal axis represents elapse time (week) from the administration started.

Figure 9: represents transition of abdominal pain score from base line in each treatment group of female diarrhea-type IBS patients. The vertical axis represents amount of change from baseline. The horizontal axis represents elapse time (week) from the administration started.

Figure 10: represents daily responder rates in each treatment group of female diarrhea-type IBS patients. The vertical axis represents daily responder rates.

Figure 11: represents weekly responder rates in each treatment group of female diarrhea-type IBS patients. The vertical axis represents weekly responder rates.

DESCRIPTION OF THE INVENTION

Below, the present invention is described in detail.

In the present invention, the the compound of the present invention, i.e., (1S)-2-acetyl-1-(4-chloro-2-methoxyphenyl)-5-fluoro-1,2,3,9-tetrahydrospiro[β-carboline-4,1'-cyclopropane] is represented by below structural formula.

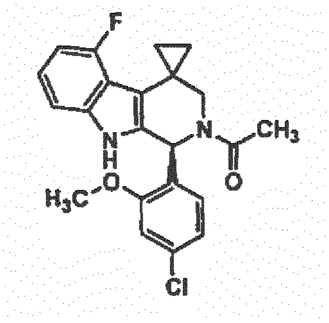

In the present invention, the the compound of the present invention can be prepared according to Example 36 (2) → Example 38 described in Patent Document 2.

In the present invention, TSPO means Translocator protein 18kDa, which is a receptor protein also referred to as MBR (Mitochondrial benzodiazepine receptor). In particular, in the present invention, a human TSPO is preferable.

In the present invention, radiotracer, which also referred to as radioligand and radioactive probe, means a radiotracer which can bind to TSPO. Examples of the radiotracer used in the present invention include [$^3$H] PK11195, [$^{11}$C] PBR28 and [$^{11}$C] DPA713. Here, as can be easily understood by a person skilled in the art, PK11195 means 1-(2-Chlorophenyl)-N-methyl-N-(1-methylpropyl) isoquinoline-3-carboxamide (CAS No. 85532-75-8), PBR28 means N-(2-methoxybenzyl)-N-[4-(phenoxy)pyridin-3-yl) acetamide (CAS No. 253307-65-2), and DPA713 means N,N-diethyl-2-[2-(4-methoxyphenyl)-5,7-dimethylpyrazolo[1,5-a]pyrimidine-3-yl] acetamide (CAS No.386297-97-8). As a radiotracer in the present invention, [$^{11}$C] PBR28 is preferable.

In the present invention, TSPO occupancy means brain TSPO occupancy of the the compound of the present invention, which was calculated by using the amount of radiotracer binding to brain TSPO before the administration of the the compound of the present invention and after the administration of the the compound of the present invention, when a radiotracer was administered.

In the present invention, based on the Biological example 8, the doses of the the compound of the present invention to be administered to a patient for preventing and/or treating stress diseases per dose include the dose that makes TSPO occupancy in the patient with stress disease reach about 50 to about 85%, i.e. the dose that reaches about 15 to about 150 ng/mL blood (plasma) concentration.

In the present invention, based on the Biological example 9, the doses of the the compound of the present invention to be administered to a patient for preventing and/or treating stress diseases per dose include the dose that makes TSPO occupancy in the patient with stress disease reach about 50 to about 95%, i.e. the dose that reaches about 15 to about 250 ng/mL blood (plasma) concentration.

In the present invention, examples of the administration method of the the compound of the present invention for achieving the above blood (plasma) concentration include, but are not limited to, oral administration, subcutaneous administration, intravenous administration, intramuscular administration, intraperitoneal administration, transdermal administration, nasal administration, pulmonary administration and the like.

In the present invention, the dose of the compound of the present invention to achieving the above blood concentration varies in administration method. In the case of oral administration, examples of the dose that reaches about 15 to about 250 ng/mL blood concentration include doses of from about 5 to about 100 mg once daily. The dose

is preferably about 20 to about 100 mg, more preferably about 50 to about 100 mg, particularly preferably about 60 mg. Examples of the specific dose include about 5 mg, about 10 mg, about 15 mg, about 20 mg, about 25 mg, about 30 mg, about 35 mg, about 40 mg, about 45 mg, about 50 mg, about 55 mg, about 60 mg about 65 mg, about 70 mg, about 75 mg, about 80 mg, about 85 mg, about 90 mg, about 95 mg, and about 100 mg. Depending on the condition of stress diseases, the dose may be appropriately increased or decreased within the range, for example, using about 100 mg as a highest daily dose. Further, the dose may be a dose of single dose or multiple doses, for example it may be a dose when administered by dividing into 1 to 4 times per day. In the present invention, examples of the administration period for maintaining the above blood concentration, and to exert prophylactic and/or therapeutic effect of stress diseases include, but are not limited to, 1 day to 1 year, 1 day to 6 months, 1 day to 3 months, 1 day to 2 months, 1 day to 1 month, 1 day to 3 weeks, 1 day to 2 weeks and 1 day to 1 week.

In the present invention, examples of stress disease include irritable bowel syndrome, functional gastrointestinal disorders, peptic ulcer, gastric and duodenal ulcer, biliary dyskinesia, esophageal spasm, stomach atony, aerophagia, chronic gastritis, chronic hepatitis, chronic pancreatitis, eating disorders (e.g. anorexia nervosa, apastia and bulimia, etc), neurogenic (psychogenic) vomiting disease, neurogenic (psychogenic) nausea disease, inflammatory bowel disease (e.g. ulcerative colitis and Crohn's disease), abdominal tense disease, gastrointestinal neurosis (e.g. belly ringing fear, etc.), anxiety related diseases, neurosis, panic disorder, sleep disorder, depression, reactive depression, epilepsy, Parkinson's disease, Parkinson's syndrome, schizophrenia, autonomic ataxia, Huntington's disease, Alzheimer's disease, affective disorder, cognitive disorder, migraine, tension type headache, cluster headache, post traumatic stress disorder (PTSD), dissociative disorder, insomnia, nervous cough, psychogenic convulsive attack, psychogenic syncopal attack, maladjustment to job, burnout syndrome, chronic fatigue syndrome (myalgic encephalomyelitis), hyperventilation syndrome, premenstrual syndrome, writer's cramp, spasmodic torticollis, chronic diarrhea, chronic constipation and gastroesophageal reflux disease. In the present invention, preferred examples of stress disease include irritable bowel syndrome, depression and anxiety related diseases.

In the present invention, examples of functional gastrointestinal disorder include functional gastroenteropathy (functional dyspepsia), functional abdominal pain syndrome and functional heartburn.

In the present invention, examples of anxiety related disease include neurosis, generalized anxiety disorder (GAD), social anxiety disorder (SAD), panic disorder, hyperactivity disorder, attention deficit, personality disorder, bipolar disorder, autism and the like. Preferred examples of anxiety related disease include panic disorder.

[Toxicity]

Toxicity of the compounds of the present invention is sufficiently low, and can be used safely as a medicine.

[Application to medicine]

Since the compound of the present invention has a TSPO antagonist effect, it is useful as a prophylactic and/or therapeutic agent for stress diseases.

The compound of the present invention may be administered as a combination drug which is combined with other drug for the purpose of (1) the complementation and/or enhancement of a prophylactic and/or therapeutic effect of the compound of the present invention for above stress diseases, (2) the improvement of pharmacokinetics/absorption of the compound of the present invention and the reduction of the dose of the compound of the present invention, and/or (3) the reduction of adverse side effects of the compound of the present invention.

The combination drug of the compound of the present invention with other drug may be administered in a form of a compounding agent in which both ingredients are incorporated into one preparation, or may take a form of administration of separate preparations. When administered by formulating into separate preparations, administration by simultaneous administration and time lag is included. In addition, in administration of time lag, the compound of the present invention may be administered earlier, and other drug may be administered later, or other drug may be administered earlier, and the compound of the present invention may be administered later. Each method of administration may be the same or different.

Examples of the other drug may be used in combination with the compound of the present invention include benzodiazepine antianxiety drug, thienodiazepine antianxiety drug, non-benzodiazepine antianxiety drug, CRF antagonist, neurokinin-1 (NK1) antagonist, tricyclic antidepressant, tetracyclic antidepressant, monoamine oxidase (MAO) inhibitor, triazolopyridine antidepressant, serotonin and noradrenaline reuptake inhibitor (SNRI), selective serotonin reuptake inhibitor (SSR1), serotonin reuptake inhibitor, noradrenergic and specific serotonergic antidepressant (NaSSA), noradrenaline and dopamine disinhibition drug (NDDI), selective serotonin reuptake enhancer (SSRE), N-methyl-D-aspartate (NMDA) receptor inhibitor, glycine transporter inhibitor, dopamine precursor, dopamine receptor agonist, catechol-O-methyl transferase (COMT) inhibitor, cholinesterase inhibitor, neurotensin antagonist, anticholinergic agent, serotonin-dopamine antagonist, central nervous system stimulant, antiepileptic drug, antivertigo drug, gastrointestinal

function adjustment drug, histamine $H_2$ receptor antagonist, proton pump inhibitor, muscarinic receptor antagonist, defense factor enhancing drug, prostaglandin derivative, opioid agonist, 5-HT4 agonist, 5-HT3 antagonist, chloride channel activator, guanylate cyclase inhibitor, bulk laxative, salt-based laxative, irritant laxative, affinity poly acrylic resin, opioid $\mu$ receptor agonist and opioid $\delta$ receptor antagonist, tryptophan hydroxylase inhibitor and antibiotics.

In the present invention, examples of benzodiazepine antianxiety drug include alprazolam, oxazepam, oxazolam, cloxazolam, clorazepate dipotassium, chlordiazepoxide, diazepam, tofisopam, triazolam, prazepam, fludiazepam, flutazolam, flutoprazepam, bromazepam, mexazolam, medazepam, ethyl loflazepate and lorazepam.

In the present invention, examples of thienodiazepine antianxiety drug include etizolam and clotiazepam.

In the present invention, examples of non-benzodiazepine antianxiety drug include citric acid tandospirone and hydroxyzine hydrochloride.

In the present invention, examples of neurokinin-1 (NK1) antagonist include aprepitant and fosaprepitant meglumine.

In the present invention, examples of tricyclic antidepressant include amitriptyline hydrochloride, imipramine hydrochloride, clomipramine hydrochloride, dosulepin hydrochloride, nortriptyline hydrochloride, lofepramine hydrochloride, trimipramine maleate and amoxapine.

In the present invention, examples of tetracyclic antidepressant include maprotiline hydrochloride, mianserin hydrochloride and setiptiline maleate.

In the present invention, examples of monoamine oxidase (MAO) inhibitor include safrazine hydrochloride.

In the present invention, examples of serotonin and noradrenaline reuptake inhibitor (SNRI) include milnacipran hydrochloride, venlafaxine hydrochloride and duloxetine hydrochloride.

In the present invention, examples of selective serotonin reuptake inhibitor (SSRI) include fluvoxamine maleate, paroxetine hydrochloride, fluoxetine hydrochloride, citalopram hydrochloride, sertraline hydrochloride and escitalopram oxalate.

In the present invention, examples of scrotonin reuptake inhibitor include trazodone hydrochloride.

In the present invention, examples of noradrenergic and specific serotonergic antidepressant include mirtazapine.

In the present invention, examples of noradrenaline and dopamine disinhibition drug include agomelatine.

In the present invention, examples of selective serotonin reuptake enhancer include tianeptine.

In the present invention, examples of N-methyl-D-aspartate receptor inhibitor include memantine.

In the present invention, examples of dopamine precursor include levodopa.

In the present invention, examples of dopamine receptor agonist include bromocriptine.

In the present invention, examples of COMT inhibitor include entacapone.

In the present invention, examples of cholinesterase inhibitor include donepezil and rivastigmine.

In the present invention, examples of anticholinergic agent include trihexyphenidyl, biperiden, ipratropium bromide and mepenzolate bromide.

In the present invention, examples of serotonin-dopamine antagonist include risperidone, perospirone hydrochloride hydrate, quetiapine fumarate and olanzapine.

In the present invention, examples of antiepileptic drug include phenobarbital, phenytoin, carbamazepine, valproic acid, clonazepam, levetiracetam, topiramate and ramotorikin

In the present invention, examples of antivertigo drug include difenidol and betahistinc.

In the present invention, examples of gastrointestinal function adjustment drug include trimebutine maleate and polycarbophil calcium.

In the present invention, examples of histamine $H_2$ receptor antagonist include cimetidine, ranitidine, famotidine, nizatidine and lafutidine.

In the present invention, examples of proton pump inhibitor include omeprazole, lansoprazole and rabeprazole.

In the present invention, examples of muscarinic receptor antagonist include pirenzepine.

In the present invention, examples of defense factor enhancing drug include gefarnate, teprenone, sucralfate, aldioxa, cetraxate hydrochloride and ornoprostil.

In the present invention, examples of prostaglandin derivative include ornoprostil and misoprostol.

In the present invention, examples of opioid agonist include asimadoline and nalfurafine.

In the present invention, examples of 5-HT4 agonist include tegaserod, cisapride and mosapride citriate.

In the present invention, examples of 5-HT3 agonist include ramosetron, alosetron and cilansetron.

In the present invention, examples of chloride channel activator include lubiprostone.

In the present invention, examples of guanylate cyclase inhibitor include linaclotide.

In the present invention, examples of bulk laxative include methylcellulose, carmellose and lactulose.

In the present invention, examples of salt-based laxative include magnesium sulfate and magnesium oxide.

In the present invention, examples of irritant laxative include picosulfate, lactulose, castor oil, senna and rhubarb.

In the present invention, examples of affinity poly acrylic resin include polycarbophil calcium.

In the present invention, examples of opioid μ receptor agonist and opioid δ receptor antagonist include eluxadoline.

In the present invention, examples of tryptophan hydroxylase inhibitor include LX1033.

In the present invention, examples of antibiotics include rifaximin.

EXAMPLES

Hereinbelow, the present invention will be described in detail with reference to examples. However, the present invention is not intended to be limited to these examples.

[Examples]

Biological examples were shown below. Based on these experimental methods, the present inventors confirmed the effects of the compound of the present invention.

Biological example 1:

Study of antistress effect and ex vivo brain occupancy in rats

Male Sic: Wistar rats (Japan SLC, Inc., at the time of use: 11 weeks old) was exposed to physical and mental stressors (Journal of Pharmacological Science, Vol.104, p.263-273, 2007). The vehicle (0.5 w/v% methylcellulose 400cP solution) or the compound of the present invention (0.1, 0.3, 1 and 3 mg / kg) was orally administered. Two hours after the administration, the stressor load was started by putting the rat in a restraint stress cage (Natsume Seisakusho Co., Ltd.). The antistress effect was evaluated by measuring the stool weight which was excreted during the stressor load of 1 hour (n=12 in each group). As compared to the stool amount of the normal group that does not load the stressor (stool wet weight: average 0.04 g), remarkable evacuation was observed in the vehicle treated group that loaded the stressor (stool wet weight: average 1.79 g). Since the average stool wet weight was 1.48, 0.96, 0.60 and 0.67 g in each 0.1, 0.3, 1 and 3mg/kg treated group of the compound of the present invention, it turned out that the compound of the present invention significantly inhibit the stool wet weight at the dose of 0.3, 1 and 3 mg/kg compared to the vehicle treated group.

Next, brain TSPO occupancy of the compound of the present invention was measured by using brain (cerebral cortex and hippocampus) homogenates of the vehicle treated group and the compound of the present invention treated group, using [$^3$H] PK11195 as a radiotracer. PK11195 was dissolved in dimethyl sulfoxide (DMSO) to prepare a 4 mmol/L solution. After preparing an ethanol solution of 100 nmol/L [$^3$H] PK11195 (PerkinElmer, Inc.), the solution was diluted 50-fold with 50 mmol/L HEPES buffer (Sigma-Aldrich Co.). In the case of determing the total binding amount of the radiotracer, 50 mmol/L HEPES (99 μL) and DMSO (1 μL) were added to Nunc MiniSorp (trade name, 12 × 75mm, Thermo Fisher Scientific Inc.). In the case of determing the nonspecific binding amount of the radiotracer, 4 mmol/L PK11195 (1 μL) was substituted for DMSO (1 μL). The brain homogenate (50 μL) derived from cerebral cortex or the hippocampus was added to the tube. Then [$^3$H] PK11195 (50 μL) (cerebral cortex: final concentration 0.4532 nmol/L, hippocampus: final concentration 0.4520 nmol/L) was added and stirred, and the mixture was incubated for 90 minutes at 4°C. After completion of the reaction, ice-cold 50 mmol/L HEPES (2 mL) was added to the tube, and then the mixture was suction filtered on a glass fiber filter (GF/B, Brandel Inc.) which pretreated with 0.3 w/v% polyethyleneimine (Sigma-Aldrich Co.), to collect the brain homogenate. After drying the glass fiber filter, the absorbing part of the brain homogenate was transferred to a liquid scintillation vial. ACS-II scintillation cocktail (7 mL) was added to the liquid scintillation vial and stirred, and the radioactivity was measured by liquid scintillation counter (TRI-CARB2900TR, PerkinElmer Life and Analytical Sciences Inc.). Specific binding amount (dpm) of [$_3$H] PK11195 in each brain homogenate was set to a value obtained by subtracting the nonspecific binding amount (dpm) from the total binding amount (dpm). TSPO occupancy (%) in cerebral cortex and hippocampus, of the compound of the present invention treated group, were calculated according to the following equation.

$$\text{TSPO Occupancy (\%)} = (1 - X/Y) \times 100$$

X: Specific binding amount (dpm) in each brain homogenate

Y: Average value (dpm) of specific binding amount in control group

As a result, the average TSPO occupancy of 0.1, 0.3, 1 and 3 mg/kg group of the compound of the present invention was 11.8, 47.7, 83.8 and 94.5% in the cerebral cortex, or 25.7, 50.7, 86.3 and 95.1% in the hippocampus.

Biological example 2:

Study of antidepressant effect

Male Crlj: WI rats (Charles River Laboratories Japan, Inc., at the time of surgery: 11 weeks old) were used to prepare an olfactory bulb isolated rat in accordance with Yamaguchi et al. method (Journal of the Pharmaceutical Society of Japan, Vol.130, p.175-183, 2007). The head of the anesthetized rat with pentobarbital sodium was fixed to brain stereotaxic apparatus (ASI Instruments Inc., NARISHIGE). After incising the scalp, holes to the left and right olfactory bulb region of the skull (7 mm forward and 1.8 mm laterally from bregma) were made with a dental drill. After aspirating and removing the left and right olfactory bulb by an aspirator coupled to an oral sonde which except for the tip, scalp was sutured. After the surgery, the rat was put in a stainless steel five series cage, wherein the individual breeding space was partitioned in half by a black acrylic plate, to breed the rat in an isolated condition, wherein neighboring rats are invisible. Also, completely cover the breeding shelf for put the stainless steel five series cage using a black plastic sheet for breeding the rat in a dark state. The rat of the sham surgery group was fixed the head under anesthesia, was incised the scalp and was made a hole to the left and right olfactory bulb region of the skull, without the suction and remove of the olfactory bulb, was sutured the scalp. And the isolation and segregation breeding was not conducted. After 14 days of surgery, the excessive emotional reactivity was evaluated in accordance with the method of Gomita et al. (Journal of the Pharmaceutical Society of Japan, Vol.82, p.267-292,1983). The animal of total score 20 points was used for the experiment.

Evaluation criteria of excessive emotional reactivity

A. Reactions to a bar that was held out to front of the nose

0: no reaction
1: interest to the target
2: defense or escape behavior to the target
3: aggressive behavior such as biting
4: violent aggressive behavior

B. Reactions to a spraying air

0: no reaction
1: only slightly move the body
2: startle response
3: show a prominent startle response, but do not jump
4: show a prominent startle response, and jump

C. Resistances to capture or handling

0: no resistance, prominent muscle relaxation
1: easy to capture or handling
2: easy to capture or handling, with slight muscle tension
3: with muscle tension, difficult to capture or handling
4: extremely difficult to capture and remarkable muscle tension

D. Reactions when clasp the tail with forceps

0: no reaction
1: interest to the target
2: defense or escape behavior to the target
3: aggressive behavior such as biting
4: violent aggressive behavior

E. Cries during the test (A to D)

0: not cry at all
1: cry sometimes
2: cry violently

A vehicle (physiological saline solution containing 0.5% Tween 80) or the compound of the present invention (0.3, 1 or 3 mg/kg) was orally administered to the olfactory bulb isolated rat repeatedly once daily for 8 days. The vehicle was administered to the rat in the sham surgery group. On day 7, the excessive emotional reactivity was evaluated before and 1 hour after the administration.

As shown in Figure 1 (## means $p<0.01$ vs. sham surgery group at Wilcoxon rank sum test, ** means $p<0.01$ vs. vehicle control group at Steel test.), the excessive emotional score was significantly higher in the vehicle control

group wherein the isolation and segregation breeding was conducted after olfactory bulb isolation compared with the sham surgery group at the time of before to divide the group, before and after the administration on day 7. On the other hand, the excessive emotional score was significantly lower in the compound of the present invention (0.3, 1 and 3 mg/kg) repeatedly orally administered group compared with the vehicle control group at the time of before and after the administration on day 7. From this result, the compound of the present invention was found to have an antidepressant effect.

Biological example 3:

Study of antianxiety effect (1)

Male Crj: CD (SD) rats (Charles River Laboratories Japan, Inc., at the time of evaluation: 8 weeks old) were used to load conditioned fear stress in accordance with Funatsu et al. method (European Journal of Pharmacology, Vol.573, p.190-195, 2007). The day before of fear conditioning, the rat was put in an electric shock device which was placed in a soundproof box to acclimate to the evaluation environment. The day of fear conditioning, the rat was put in the electric shock device. The conditioning, 5 seconds electric current load (2.0 mA) and light irradiation (3 pieces of 40 W fluorescent bulbs) followed by 3 seconds warning sound (60 to 70 db), was carried out a total 15 times at 1 minute intervals. The rat of the non-stressed group was given a warning sound and light irradiation without performing the electric current load during fear conditioning. The day after the fear conditioning, the vehicle (0.5 w/v% methylcellulose 400cP solution) or the compound of the present invention (0.3, 1 or 3 mg/kg) was orally administered to the rat. Two hours after the administration, the rat was put in the electric shock device. A stress load, only 5 seconds light irradiation followed by 3 seconds warning sound, was carried out on the rat a total 30 minutes at 1 minute intervals. The behavior of the rat was photographed with a video camera during the 30 minites of stress load and freezing time was measured.

As a result, as shown in Figure 2 (### means $p<0.001$ vs. non-stressed group at t-test, * means $p<0.05$ vs. vehicle control group at Dunnett test), the freezing time was longer in the vehicle control group wherein the vehicle was administrated to the stress load rat compare with the non-stressed group. The compound of the present invention was shortened this extended freezing time significantly at the dose of 1 and 3 mg/kg. From this result, the compound of the present invention was found to have an antianxiety effect.

Biological example 4:

Study of antianxiety effect (2)

Male LEW/CrlCrlj rats (Charles River Laboratories Japan, Inc., at the time of evaluation: 6 weeks old) were used. In accordance with Rupprecht et al. method (Science, Vol. 325, p.490-493, 2009), cholecystokinin tetrapeptide (CCK-4) (3 mg/kg) was administred subcutaneously to the rat to induce freezing behavior which was used as an indicator of anxiety.

The vehicle (0.5 w/v% methylcellulose 400cP solution) or the compound of the present invention (1 or 3 mg/kg) was orally administered to the rat. Two hours after the administration, CCK-4 (3 mg/kg) was subcutaneously administered.Two minutes after the administration ofCCK-4, the front paws of the rat were placed on a wooden block of height 7 cm. The freezing time, wherein the rat does not move at all other than a motion due to breathing, was measured by stopwatch. Instead of CCK-4, the normal group was administered 1% dimethyl form amide which is the vehicle of CCK-4. Cut-off time was set to 120 seconds. This trial was carried out a total five times at about 2 minute intervals, and the sum of the freezing time was calculated.

As a result, as shown in Figure 3 (### means $p<0.001$ vs. normal group at Wilcoxon rank sum test, * means $p<0.05$ vs. vehicle control group at Steel test, ** means $p<0.01$ vs. vehicle control group at Steel test, *** means $p<0.001$ vs. vehicle control group at Steel test), the freezing time was longer in the vehicle control group wherein the vehicle was administered to the stress load rat compared with the normal group. The compound of the present invention was shortened this extended freezing time significantly at the dose of 1 and 3 mg/kg. From this result, the compound of the present invention was found to have an antianxiety effect.

From the above mentioned results of Biological examples 1 to 4, about 50% or more brain TSPO occupancy that can be achieved by the administration of 0.3 to 3 mg/kg of the compound of the present invention is required for the compound of the present invention exert an antistress effect, an antidepressant effect and an antianxiety effect. And the maximal effects were found to be achieved by about 85% to about 95% brain TSPO occupancy from the fact that the efficacy at the dose of 1 mg/kg to 3 mg/kg in the disease models in rats has almost reached a plateau.

Biological example 5:

Comparison of radiotracer in rat ex vivo brain occupancy

The vehicle (0.5 w/v% methylcellulose 400cP solution) or the compound of the present invention (0.03, 0.1, 0.3, 1 or 3 mg/kg) was orally administered to male S1c: Wistar rats (Japan SLC, Inc., at the time of use: 11 weeks old). After 3 hours of the administration, in the same manner as Biological example 1, brain TSPO occupancy of the compound of the present invention relative to the specific binding of the vehicle control group was measured by measuring the specific binding amount of [$^3$H] PK 11195 or [$^3$H] PBR28 (Sekisui Medical Co., Ltd.) of brain (cerebral cortex) homogenates (n=8 in each group). As a result, the average TSPO occupancy of 0.03, 0.1, 0.3, 1 and 3 mg/kg group of the compound of the present invention was -1.3, 14.9, 48.1, 80.9 and 91.3% in the cerebral cortex when [$^3$H] PK11195 was used as a radiotracer. On the other hand, the average TSPO occupancy was 6.5, 11.5, 40.4, 77.7 and 90.2% in the cerebral cortex when [$^3$H] PBR28 was used as a radiotracer. From the above, TSPO occupancy of the compound of the present invention was found to be comparable in the both case of using [$^3$H] PBR28 or [$^3$H] PK11195 radiotracer.

Biological example 6:

Study of ex vivo brain occupancy in monkeys

The vehicle (0.5 w/v% methylcellulose 400cP solution) or the compound of the present invention (0.4 or 1 mg/kg) was orally administered twice daily for 3 days, once on day 4 to male rhesus monkeys (Shin Nippon Biomedical Laboratories, Ltd., at the time of use: 4 to 6 kg). After 2 hours of the final administration, in the same manner as Biological example 1, brain TSPO occupancy of the compound of the present invention relative to the specific binding of the vehicle control group was measured by measuring the specific binding amount of [3H] PBR28 on brain (anterior cingulate cortex, caudate nucleus, amygdala, hippocampus and occipital lobe) homogenates (n=3 in vehicle control group, n=5 in each the compound of the present invention treated group). As a result, the average TSPO occupancy of brain 5 sites was 37.9 and 56.6% in 0.4 and 1 mg/kg group of the compound of the present invention. The average plasma concentration of the compound of the present invention was 23.9 and 154 ng/mL in 0.4 and 1 mg/kg group.

Biological example 7:

Study of brain occupancy using PET in monkeys

Male rhesus monkeys (Hamri Co., Ltd., at the time of use: 5 to 8 kg) were used. The vehicle (0.5 w/v% methylcellulose 400cP solution) or the compound of the present invention (0.3 to 30 mg/kg) was once orally administered to 3 rhesus monkeys. After 2 hours of the administration, a PET measurement using [$^{11}$C] PBR28 was performed in the following manner.
Measurement equipment: PET camera; SHR-7700 (Hamamatsu Photonics K.K.)
Image reconstruction: SHR Control II program (Hamamatsu Photonics K.K.)
Change over time in brain radioactivity concentration and calculation of distribution volume: PMOD programs (PMOD Technologies Ltd.)
Measurement methods: 120 minutes blank measurement and 30 minutes transmission measurement were performed using 68Ge/68Ga calibration source. Next, [$^{11}$C] PBR28 was intravenously bolus administered over a period of about 30 seconds. Emission measurement (total 121 minutes, 55 frames) was started together with the administration while an arterial blood sampling for measuring plasma concentration.
The compound of the present invention (0.3 to 10 mg/kg) was orally administered twice daily for 3 days, once on day 4 to 3 rhesus monkeys. After 2 or 24 hours of the final administration, a PET measurement was carried out using [$^{11}$C] PBR28. Brain TSPO occupancy relative to the distribution volume of the vehicle control group was calculated by calculating the distribution volume of the interest area (cerebellum, hippocampus, striatum, thalamus, occipital lobe, temporal lobe, frontal lobe and parietal lobe) from the brain radioactivity concentration time change of [$^{11}$C] PBR28 and arterial blood plasma radioactivity which was corrected in a non-metabolic rate. As shown in Figure 4, it was found that brain TSPO occupancy depending on the plasma concentration of the compound of the present invention within each individual by analyzing the relationship between brain TSPO occupancy and the plasma concentration of the compound of the present invention on each individual. As a result, it was found that brain TSPO occupancy reaches about 50% when the plasma concentration of the compound of the present invention is about 70 ng/mL. Further, from this result, it was turned out that there is no difference in the relationship between TSPO occupancy and plasma concentration which was measured in ex vivo (Biological example 6) and the relationship between TSPO occupancy and plasma concentration which was measured in PET.

Biological example 8:

Study of brain occupancy using PET in humans (1)

Male and female healthy adults were used. The compound of the present invention (6 to 200 mg) was once orally administered. Before and after 24 hours of the administration, a PET measurement using [$^{11}$C] PBR28 was performed in the following manner.
Measurement equipment: PET; ECAT HR + (Siemens)
Measurement methods: 90 minutes blank measurement and 10 to 15 minutes transmission measurement were performed using 68Ge/68Ga calibration source. Next, [$^{11}$C] PBR28 was intravenously bolus administrated over a period of about 30 seconds. Emission measurement (total 90 minutes, 26 frames) was started together with the administration while an arterial blood sampling for measuring the plasma concentration.
Brain occupancy, relative to the binding potential (BP) before the administration, was calculated by calculating the BP of each interest area from the brain radioactivity concentration time change of [$^{11}$C] PBR28, nonspecific binding amount and arterial blood plasma radioactivity which was corrected in a non-metabolic rate (n=3 to 4 in each group). Further, the average TSPO occupancy of all interest areas was determined from the Lassen plot that obtained by the horizontal axis of the distribution volume before administration and the vertical axis of the value obtained by subtracting the distribution volume after administration from the distribution volume before the administration for each interest area. Further, as shown in Figure 6, the Emax model was used in order to analyze the relationship between blood concentration and brain TSPO occupancy of the compound of the present invention in each individual. As parameters in this model, Emax (a maximal effect) and EC50 (ng/mL) (a blood concentration which can be achieved 50% Emax) were used. An analysis software NONMEM7.1.2 (ICON Development Solutions) was used in this analysis. Parameters and formula of Emax model were shown in the following.

$$\text{TSPO Occupancy (\%)} = (\theta 1 \times X) / (\theta 2 + X)$$

$$\theta 1\ (=\text{Emax}) = 92.8 \pm 3.59$$

$$\theta 2\ (=\text{EC50}) = 13.7 \pm 3.74$$

X = Plasma concentration (ng/mL)
As a result, the plasma concentration that makes brain TSPO occupancy reach about 50% was about 15 ng/mL. Likewise, the plasma concentration that makes brain TSPO occupancy reach about 85% was about 150 ng/mL.
Further, a transition in the plasma concentration of the compound of the present invention, in a steady state when administered once daily, was predicted. And brain TSPO occupancy was predicted by using the relationship between plasma concentration and brain TSPO occupancy that shown in Figure 5. As a result, it was found that the administration dose of the compound of the present invention is about 5 mg to about 100 mg daily in order to achieve about 50 to about 95% TSPO occupancy, which is the minimum value in the steady state.

[Table 1]

| | ROmax (%) | | | | ROmin (%) | | |
|---|---|---|---|---|---|---|---|
| Dose (mg) | Predicted 5 percentile | Predicted median | Predicted 95 percentile | | Predicted 5 percentile | Predicted median | Predicted 95 percentile |
| 1 | 28.3 | 38.4 | 48.9 | | 11.5 | 19.4 | 30.5 |
| 5 | 60.5 | 72.0 | 84.6 | | 38.0 | 52.5 | 67.7 |
| 10 | 69.6 | 80.9 | 94.2 | | 51.8 | 67.1 | 81.0 |
| 20 | 75.1 | 86.4 | 100.0 | | 646 | 77.6 | 91.5 |
| 30 | 77.0 | 88.5 | 102.6 | | 69.7 | 81.8 | 94.9 |
| 50 | 78.7 | 90.2 | 104.0 | | 74.1 | 85.8 | 99.0 |
| 60 | 78.8 | 91.0 | 104.2 | | 75.0 | 87.0 | 100.1 |

(continued)

| | ROmax (%) | | | | ROmin (%) | | |
|---|---|---|---|---|---|---|---|
| Dose (mg) | Predicted 5 percentile | Predicted median | Predicted 95 percentile | | Predicted 5 percentile | Predicted median | Predicted 95 percentile |
| 70 | 79.4 | 91.2 | 104.8 | | 76.4 | 88.2 | 101.1 |
| 100 | 80.1 | 92.1 | 106.1 | | 77.6 | 90.0 | 103.1 |

Dose (mg): dose of the compound of the present invention (mg)
ROmax (%): maximum value (%) of brain TSPO occupancy (RO)
ROmin (%): minimum value (%) of brain TSPO occupancy (RO)
Predicted 5 percentile: predicted 5% value
Predicted median: predicted median value
Predicted 95 percentile: predicted 95% value

From the above mentioned results of Bbiological example 1 to Biological example 8, TSPO occupancy which shows antistress effect, antidepressant effect and antianxiety efferc was found to be about 50 to about 95% in rats. And then the relationship between TSPO occupancy that can shows antistress effect and plasma concentration was also found in monkeys. In addition, because [$^{11}$C] PBR28 was functional as a radiotracer for TSPO in humans, the relationship between TSPO occupancy and plasma concentrations of the compound of the present invention in humans was found for the first time. And a species difference between monkeys and humans in the correlation between TSPO occupancy and plasma concentration was also found.

Biological example 9:

Study of brain occupancy using PET in humans (2)

The data in Biological example 8 were analyzed in a different analytical method. Brain occupancy, relative to the binding potential (BP) before administration, was calculated by calculating the BP of each interest area from the brain radioactivity concentration time change of [$^{11}$C] PBR28, nonspecific binding amount and arterial blood plasma radioactivity which was corrected in a non-metabolic rate (n=3 to 4 in each group). Further, the average TSPO occupancy of all interest areas was determined from the Lassen plot that obtained by the horizontal axis of the distribution volume before administration (Vt baseline) and the vertical axis of the value obtained by subtracting the distribution volume after administration from the distribution volume before the administration (Vt baseline - Vt onmed) for each interest area. Further, as shown in Figure 7, the Emax model was used in order to analyze the relationship between blood concentration and brain TSPO occupancy of the compound of the present invention in each individual. As parameters in this model, Bmax (a maximum number of binding) and Ki (ng/mL) (a blood concentration which can be achieved 50% Bmax) were used. Bmax was fixed to 1 in this analysis, and SAS Version 9.2 (SAS Institute) was used as an analysis software. Parameters and formula of Emax model were shown in the following.

$$\text{TSPO Occupancy (\%)} = \text{Bmax} \times \text{Cave} / (\text{Ki} + \text{Cave}) \times 100$$

Ki = 13.6
Cave = Mean plasma concentration (ng/mL)

A transition in the plasma concentration of the compound of the present invention, in a steady state when administered once daily, was predicted. And as shown in Table 2, brain TSPO occupancy was predicted by using the relationship between plasma concentration and brain TSPO occupancy that shown in Figure 5.

From the above mentioned results of Bbiological example 1 to Biological example 7, TSPO occupancy which shows antistress effect, antidepressant effect and antianxiety efferc was found to be about 50 to about 95% in rats. And then the relationship between TSPO occupancy that can shows antistress effect and plasma concentration was also found in monkeys. In addition, because [$^{11}$C] PBR28 was functional as a radiotracer for TSPO in humans, the relationship between TSPO occupancy and plasma concentrations of the compound of the present invention in humans was found for the first time. And a species difference between monkeys and humans in the correlation between TSPO occupancy and plasma concentration was also found. Further more, by combining the results of Biological example 9 for predict

brain TSPO occupancy in humans, it was found that the administration dose of the compound of the present invention is about 5 mg to about 100 mg daily in order to achieve about 50 to about 95% TSPO occupancy, which is the minimum value in the steady state.

[Table 2]

| Dose (mg) | TSPO Occupancy (%) | | |
|---|---|---|---|
| | Predicted 5 percentile | Predicted median | Predicted 95 percentile |
| 5 | 42.8 | 57.4 | 70.3 |
| 10 | 59.8 | 73.1 | 82.3 |
| 20 | 74.6 | 84.4 | 90.5 |
| 30 | 81.6 | 89.0 | 93.4 |
| 60 | 90.0 | 94.2 | 96.7 |
| 100 | 95.3 | 97.4 | 98.4 |

Dose (mg): dose of the compound of the present invention (mg)
TSPO occupancy (%): brain TSPO occupancy (%) in trough concentration
Predicted 5 percentile: predicted 5% value
Predicted median: predicted median value
Predicted 95 percentile: predicted 95% value

Based on the effective amount set in die above, it is possible to perform an efficacy study in humans. For example, three clinical trials of the following can indicate.

Biological example 10:

Efficacy trial in humans 1

As a clinical trail, a duble-bind, prallel-goup study was conducted under the following conditions in female diarrhea-type IBS patients.
PURPOSE: Confirmation of efficacy against clinical symptoms of IBS
METHODS: This study was conducted at 49 centers in the United States and recruited 200 patients (aged 18-65 years) with female diarrhea-type IBS patients according to Rome III criteria (Gastroenterology, Vol.130, p.1480-1491, 2006). Patients were randomly assigned to any of the following three treatment groups (1:1:1 ratio) and received orally administration once-daily for 4 weeks. Patients recorded their IBS symptoms on a daily basis in an e-diary during a 2-week pre-treatment, the 4-week treatment period and for 4 weeks post-treatment. Safety monitoring was performed throughout the study. The primary endpoints were change from baseline (pre-treatment period) to week 4 in stool consistency (Bristol Stool Scale; BSS), stool frequency and abdominal pain (Numerical Rating Scale; NRS). Secondary endpoints included FDA responder analyses (Guidance for Industry Irritable Bowel Syndrome - Clinical Evaluation of Drugs for Treatment).

TREATMENT GROUPS:

(1) Placebo group
(2) The compound of the present invention (20 mg treatment) group
(3) The compound of the present invention (60 mg treatment) group

ENDPOINTS: IBS symptoms (abdominal pain, stool consistency, stool frequency, faecal urgency and severity), QOL, psychological symptoms, safety, pharmacokinetics and biomarker RESULTS: Baseline value of patient layer and each of the parameters (stool consistency, stool frequency and abdominal pain) were similar in each treatment group. Improvements over placebo treatment group in the number of days per week with at least one stool having a BSS classification of 6 or 7 and average weekly score of the worst abdominal pain experienced during the past 24 hours were observed in 60 mg treatment group of the compound of the present invention over the 4-week treatment period (Figure

8 and Figure 9). Further, a greater percentage of patients met responder criteria among patients in 60 mg treatment group of the compound of the present invention compared to placebo treatment group (Table 3, Figure 10 and Figure 11). On the other hand, the increase in the number of days per week with no abdominal pain at the 4th week of treatment period were 0.61 in placebo treatnent group, 1.16 in 20 mg treatment group and 1.51 in 60 mg treatment group. The compound of the present invention was well-tolerated and had a favourable safety profile.

The daily responder of abdominal pain was defined as a patient who showed the number of days, which the abdominal pain score was decreased 30% or more campared with baseline, was 50% or more during 4-week treatment period. The daily responder of stool consistency was defined as a patient who showed the number of days, which the stool consistency score was 5 or less in BSS or without defecation, was 50% or more during 4-week treatment period. The daily responder of abdominal pain and stool consistency was defined as a patient who showed the number of days met the both criteria (the abdominal pain score was decreased 30% or more compared with baseline and the stool consistency score was 5 or less in BSS or without defecation) was 50% or more during 4-week treatment period.

The weekly responder of abdominal pain was defined as a patient who showed the number of weeks, which the average abdominal pain score per week was decreased 30% or more compared with baseline, was 50% or more during 4-week treatment period. The weekly responder of stool consistency was defined as a patient who showed the number of days per week with at least one stool having a BSS classification of 6 or 7 was decreased 50% or more during 4-week treatment period. The weekly responder of abdominal pain and stool consistency was defined as a patient who met the both criteria of the above-mentioned abdominal pain score and stool consistency.

[Table 3]

| | Abdominal Pain and Stool Consistency | | | Abdominal Pain | | | Stool Consistency | | |
|---|---|---|---|---|---|---|---|---|---|
| | | The compound of the present invention | | | The compound of the present invention | | | The compound of the present invention | |
| | Placebo (n=46) | 20mg (n=41) | 60 mg (n=45) | Placebo (n=46) | 20mg (n=41) | 60 mg (n=45) | Placebo (n=46) | 20mg (n=41) | 60 mg (n=45) |
| Weekly responder (%) | 21.7 | 22 | 37.8 | 39.1 | 39 | 57.8 | 28.3 | 41.5 | 48.9 |
| Daily responder (%) | 6.5 | 14.6 | 20 | 37 | 36.6 | 53.3 | 10.9 | 17.1 | 26.7 |

Biological example 11:

Efficacy trial in humans 2

As a clinical trail, a duble-bind, prallel-goup study is conducted under the following conditions in female diarrhea-type IBS patients.

PURPOSE: Confirmation of efficacy against perception and movement at a time of an intestinal tract extension using barostat method

TREATMENT GROUPS: each group is orally administered once per day after meal

(1) Placebo group
(2) The compound of the present invention (60 mg treatment) group

ADMINISTRATION PERIOD: 2 weeks

ENDPOINTS: abdominal pain, a sense of urgency and bowel movement at a time of an intestinal tract expansion, IBS severity, QOL, psychological symptoms, safety, pharmacokinetics and biomarker

Biological example 12:

Therapeutic effect on stress disease (depression and anxiety-related diseases) in humans

In the clinical trial of Biological example 10, the therapeutic effect of the compound of the present invention 60 mg treatment on depression and anxiety-related diseases were evaluated using PSS (Perceived Stress Scale) (refer to Journal of Health and Social Behavior, 24, 386-396, 1983), HAM-A (Hamilton Anxiety Scale) (refer to The British journal of medical psychology, 32, 50-55, 1959) and HAM-D (Hamilton Depression scale) (refer to Journal of Neurology, Neurosurgery and Psychiatry, 23, 56-62, 1960) as rating scales. The results are shown below. Table 4, Table 5 and Table 6 shows the result of PSS score, HAM-A score and HAM-D score, respectively.

[Table 4]

| | Before treatment (baseline) | 4th week | Amount of change from baseline |
|---|---|---|---|
| Patient A | 35 | 19 | -16 |
| Patient B | 23 | 13 | -10 |
| Patient C | 18 | 6 | -12 |

[Table 5]

| | Before treatment (baseline) | 4th week | Amount of change from baseline |
|---|---|---|---|
| Patient B | 23 | 1 | -22 |
| Patient D | 22 | 4 | -18 |
| Patient E | 18 | 5 | -13 |
| Patient A | 18 | 4 | -14 |

[Table 6]

| | Before treatment (baseline) | 4th week | Amount of change from baseline |
|---|---|---|---|
| Patient F | 20 | 13 | -7 |
| Patient D | 18 | 2 | -16 |
| Patient E | 13 | 3 | -10 |
| Patient G | 13 | 4 | -9 |

From the above, an antistress effect, an antidepressant effect and an antianxiety effect of the compound of the present invention 60 mg treatment were observed, and a therapeutic effect on depression and anxiety-related diseases were suggested.

INDUSTRIAL APPLICABILITY

The present invention can bring about an attainment of about 50 to about 95% TSPO occupancy of the compound of the present invention in a patient with a stress disease and a maximal effect of the compound of the present invention. For this reason, the compound of present invention is useful as a medicament for the prevention and/or treatment of stress diseases.

**Claims**

**1.** A medicament for preventing and/or treating stress diseases, said medicament being **characterized in that** (1S)-2-acetyl-1-(4-chloro-2.methoxyphenyl)-5-fluoro-1,2,3,9-tetrahydrospiro[β-carboline-4,1'-cyclopropane] represented by formula

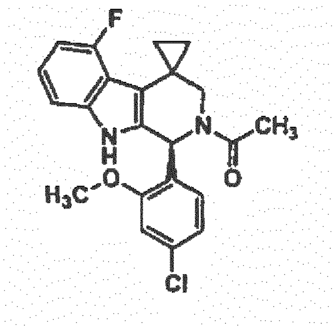

is administered at a dose that makes TSPO occupancy in the patient with stress disease reach about 50 to about 85% per dose.

2. The medicament according to claim 1, wherein the dose that makes TSPO occupancy reach about 50 to about 85% TSPO is a dose that reaches about 15 to about 150 ng/mL plasma concentration.

3. The medicament according to claim 1 or 2, wherein the adminitisration method is an oral administration.

4. The medicament according to claim 3, upon oral administration, wherein the dose that makes TSPO occupancy reach about 50 to about 85% is about 5 to about 60 mg.

5. The medicament according to any of claims 1 to 4, wherein the stress disease is irritable bowel syndrome, functional gastrointestinal disorder, depression, or anxiety-related disease.

6. The medicament according to claim 5, wherein the stress disease is irritable bowel syndrome.

7. The medicament according to any of claims 1 to 6, wherein the TSPO occupancy was measured using [$^{11}$C] PBR28 as a radiotracer.

8. A medicament for preventing and/or treating irritable bowel syndrome, said medicament being **characterized in that** (1S)-2-acetyl-1-(4-chloro-2-methoxyphenyl)-5-fluoro-1,2,3,9-tetrahydrospiro[β-carboline-4,1'-cyclopropane] represented by formula

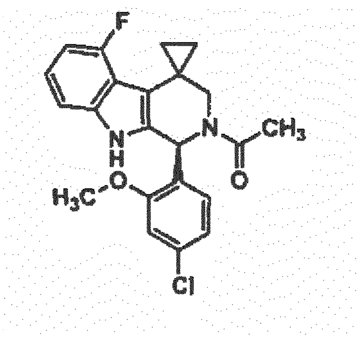

is administered orally at a dose of about 5 to about 60 mg once daily.

9. A method to set the effective dose of TSPO antagonist for preventing and/or treating stress diseases, said method being **characterized in that** [$^{11}$C] PBR28 is used as a radiotracer.

10. A method for preventing and/or treating stress diseases, wherein (1S)-2-acetyl-1-(4-chloro-2-methoxyphenyl)-5-fluoro-1,2,3,9-tetrahydrospiro[β-carboline-4,1'-cyclopropane] represented by formula

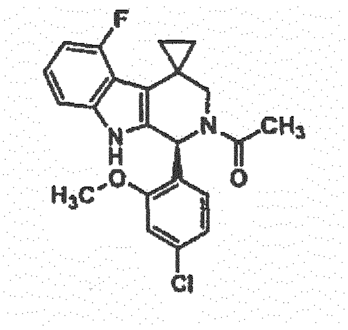

is administered to a patient with stress disease at a dose that makes TSPO occupancy in the patient with stress disease reach about 50 to about 85% per dose.

**11.** (1S)-2-acetyl-1-(4-chloro-2-methoxyphenyl)-5-fluoro-1,2,3,9-tetrahydrospiro[β-carboline-4,1'-cyclopropane] represented by formula,

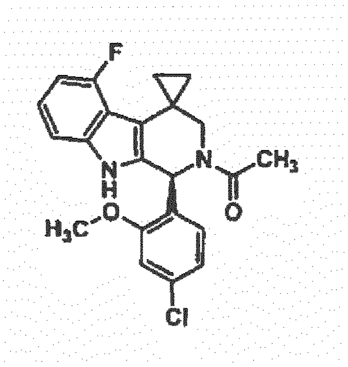

which is administered at a dose that makes TSPO occupancy in the patient with stress disease reach about 50 to about 85% per dose for preventing and/or treating stress diseases.

**12.** A medicament for preventing and/or treating stress diseases, said medicament being **characterized in that** (1S)-2-acetyl-1-(4-chloro-2-methoxyphenyl)-5-fluoro-1,2,3,9-tetrahydrospiro[β-carboline-4,1'-cyclopropane] represented by formula

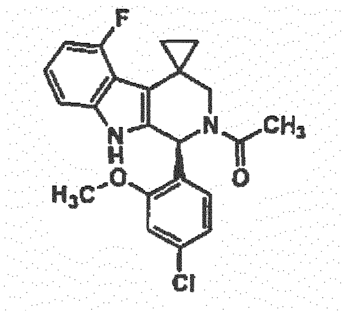

is administered at a dose that makes TSPO occupancy in the patient with stress disease reach about 50 to about 95% per dose.

**13.** The medicament according to claim 12, wherein the dose that makes TSPO occupancy reach about 50 to about 95% is a dose that reaches about 15 to about 250 ng/mL plasma concentration.

**14.** The medicament according to claim 12 or 13, wherein the adminitisration method is an oral administration.

**15.** The medicament according to claim 14, upon oral administration, wherein the dose that makes TSPO occupancy

reach about 50 to about 95% is about 5 to about 100 mg.

**16.** The medicament according to any of claims 12 to 15, wherein the stress disease is irritable bowel syndrome, functional gastrointestinal disorder, depression, or anxiety-related disease.

**17.** The medicament according to claim 16, wherein the anxiety-related disease is neurosis, generalized anxiety disorder, social anxiety disorder, panic disorder, hyperactivity disorder, attention deficit, personality disorder, bipolar disorder and autism.

Fig.1

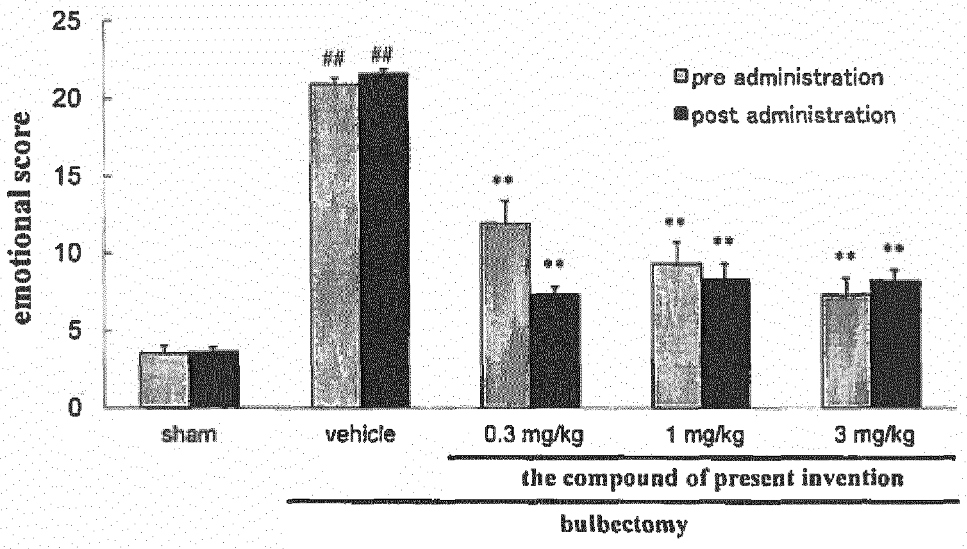
25
20
15
10
5
0
emotional score
## ##
pre administration
post administration
**
**
**
**
**
**
sham
vehicle
0.3 mg/kg
1 mg/kg
3 mg/kg
the compound of present invention
bulbectomy

Fig.2

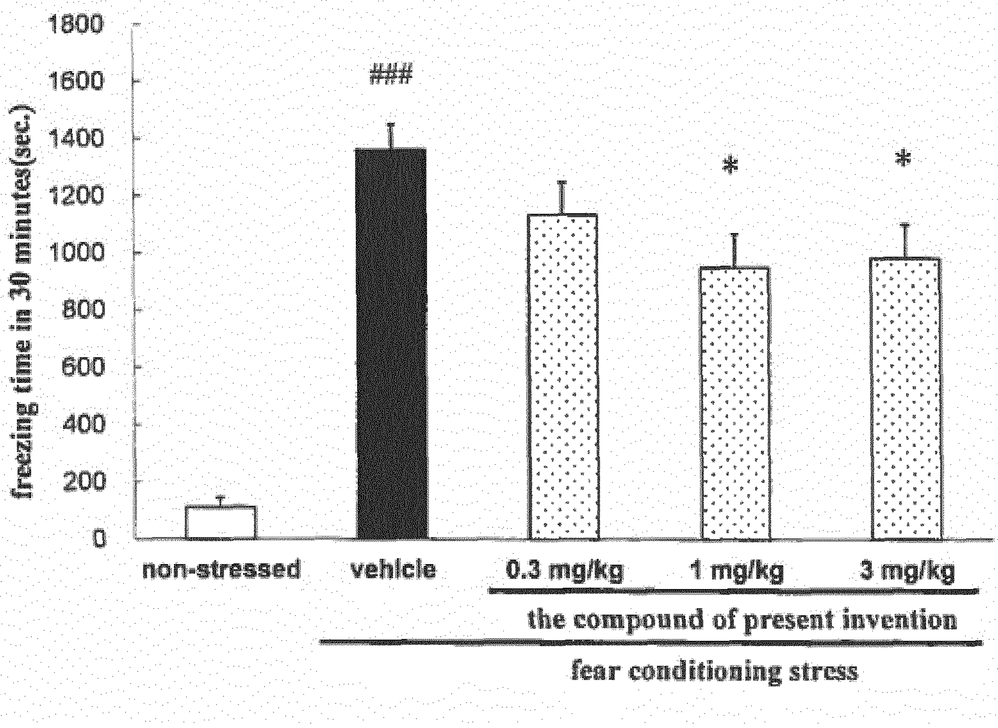
1800
1600
1400
1200
1000
800
600
400
200
0
freezing time in 30 minutes(sec.)
###
*
*
non-stressed
vehicle
0.3 mg/kg
1 mg/kg
3 mg/kg
the compound of present invention
fear conditioning stress

Fig.3

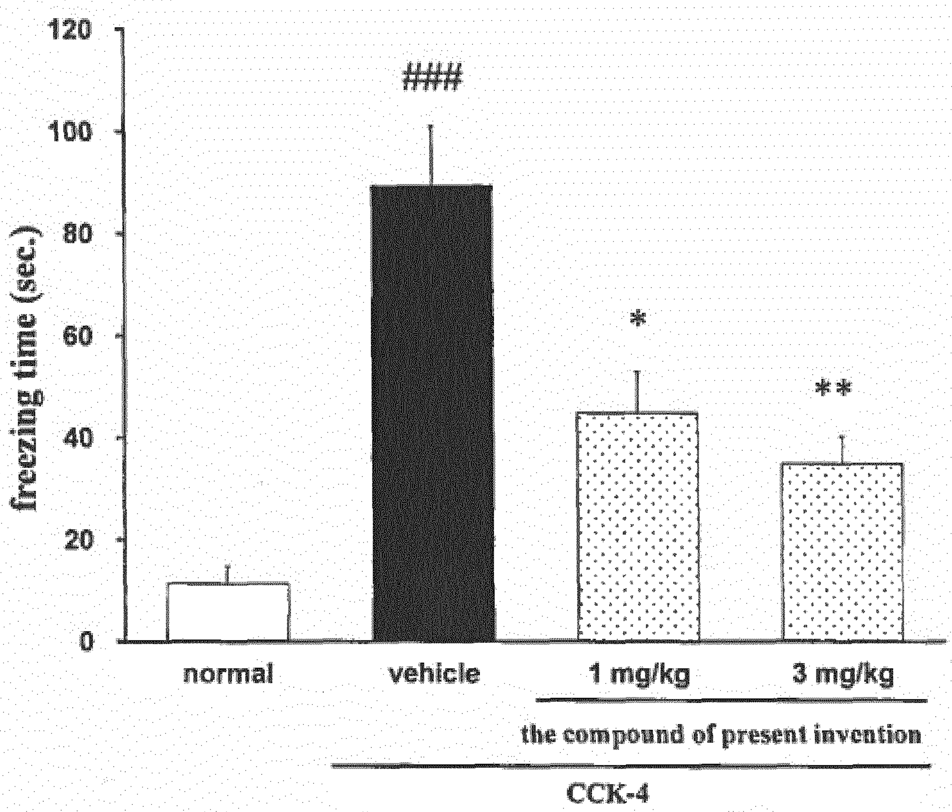
120
100
80
60
40
20
0
freezing time (sec.)
###
*
**
normal
vehicle
1 mg/kg
3 mg/kg
the compound of present invention
CCK-4

Fig.4

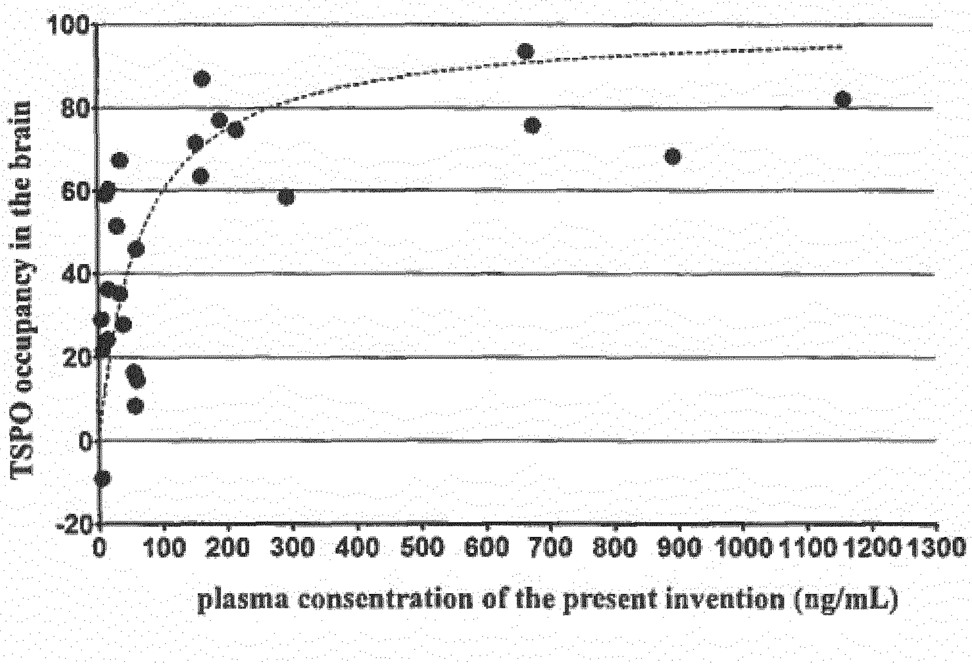
100
80
60
40
20
0
-20
TSPO occupancy in the brain
0 100 200 300 400 500 600 700 800 900 1000 1100 1200 1300
plasma consentration of the present invention (ng/mL)

Fig. 5

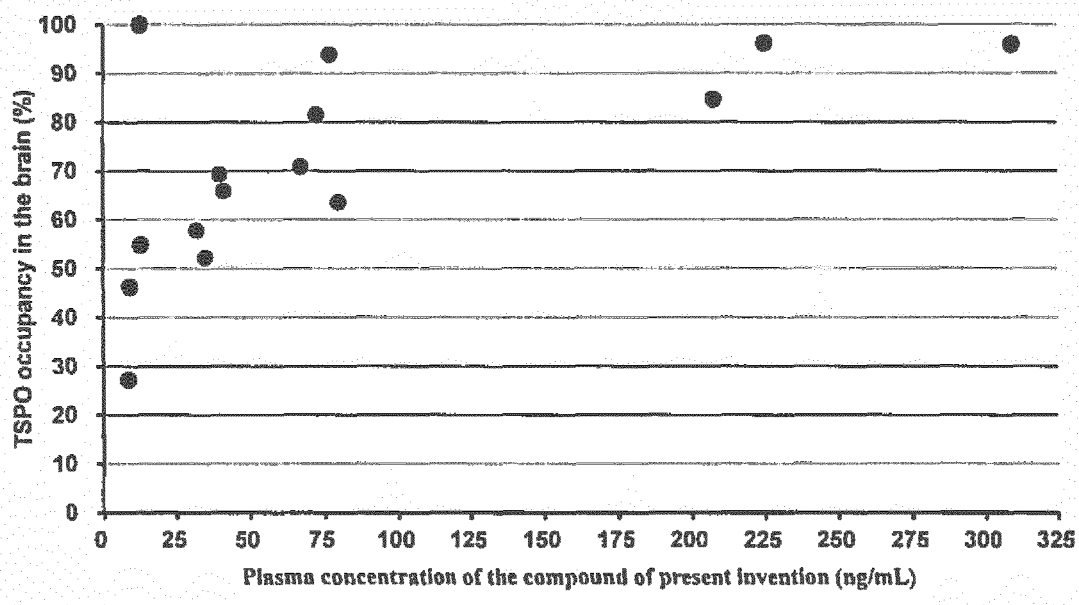
TSPO occupancy in the brain (%)
100
90
80
70
60
50
40
30
20
10
0
0 25 50 75 100 125 150 175 200 225 250 275 300 325
Plasma concentration of the compound of present invention (ng/mL)

Fig. 6

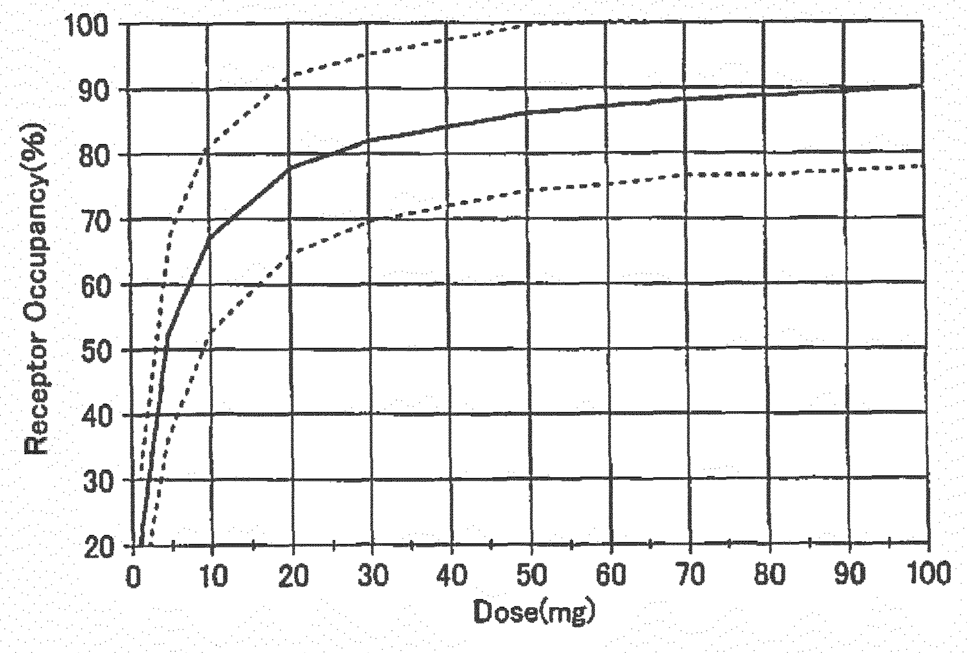
Receptor Occupancy(%)
100
90
80
70
60
50
40
30
20
0 10 20 30 40 50 60 70 80 90 100
Dose(mg)

Fig. 7

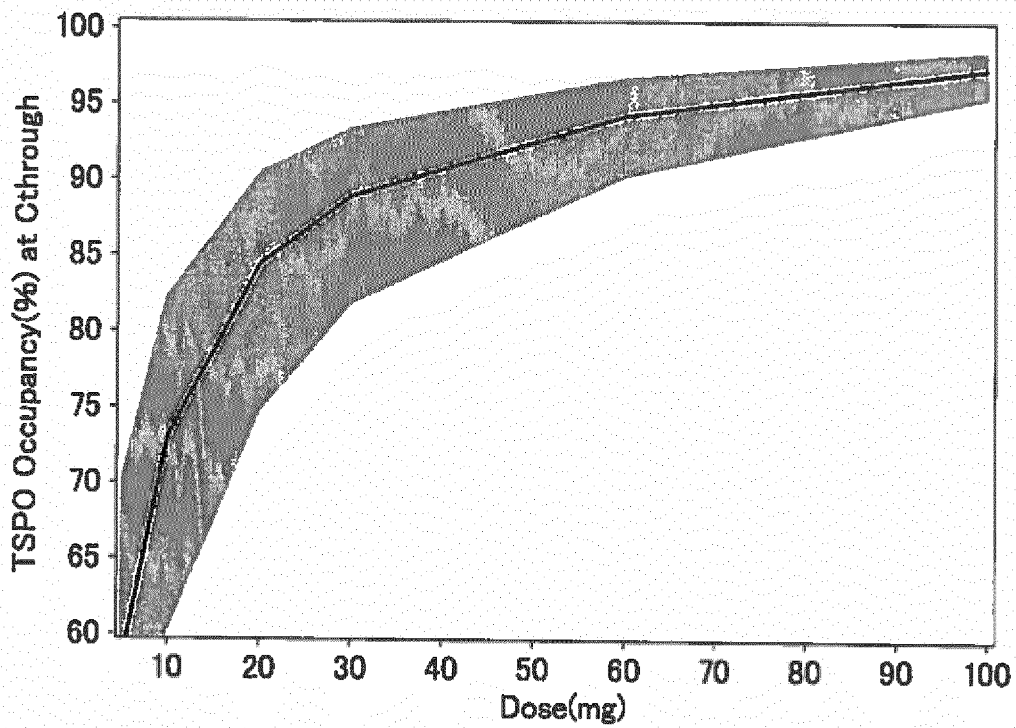
TSPO Occupancy(%) at Cthrough
100
95
90
85
80
75
70
65
60
10
20
30
40
50
60
70
80
90
100
Dose(mg)

Fig. 8

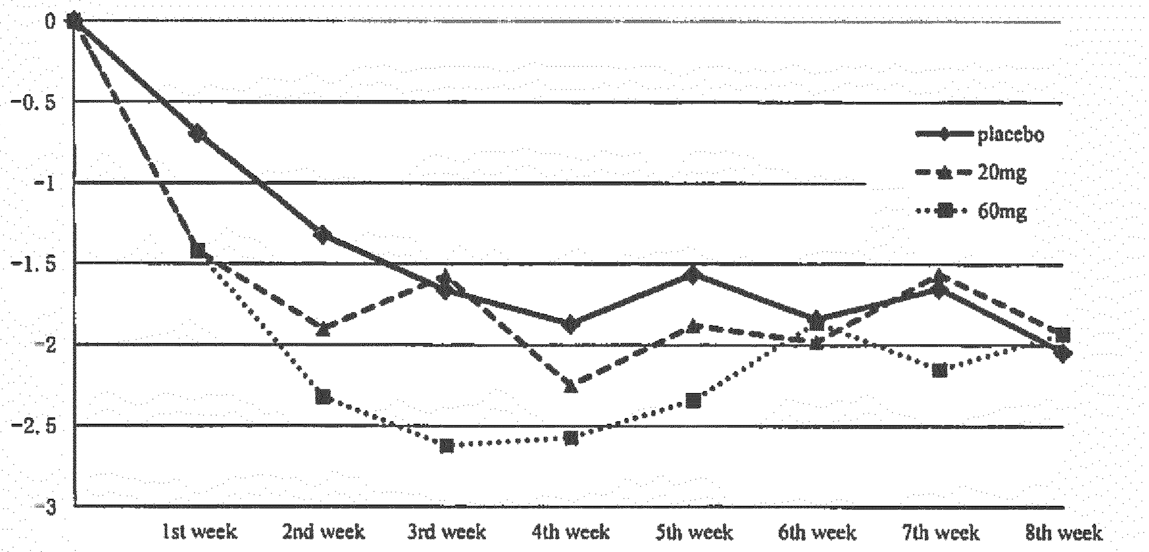
0
-0.5
-1
-1.5
-2
-2.5
-3
placebo
20mg
60mg
1st week
2nd week
3rd week
4th week
5th week
6th week
7th week
8th week

Fig.9

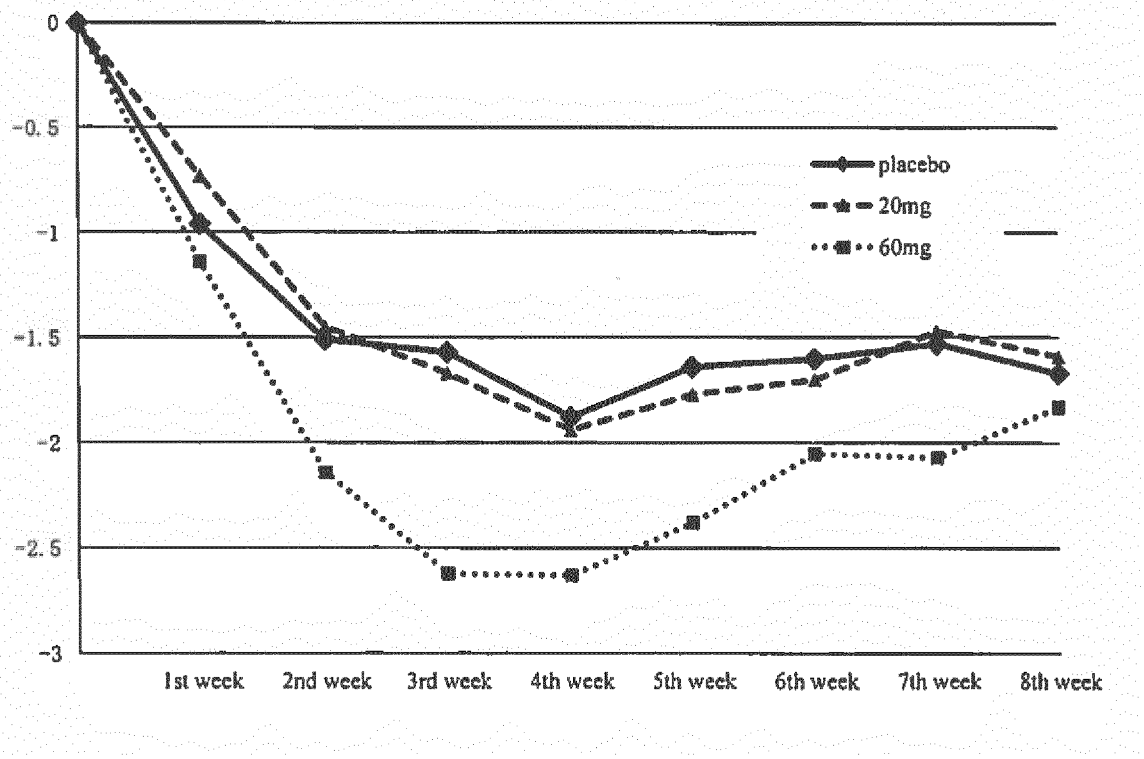
0
-0.5
-1
-1.5
-2
-2.5
-3
placebo
20mg
60mg
1st week
2nd week
3rd week
4th week
5th week
6th week
7th week
8th week

Fig.10

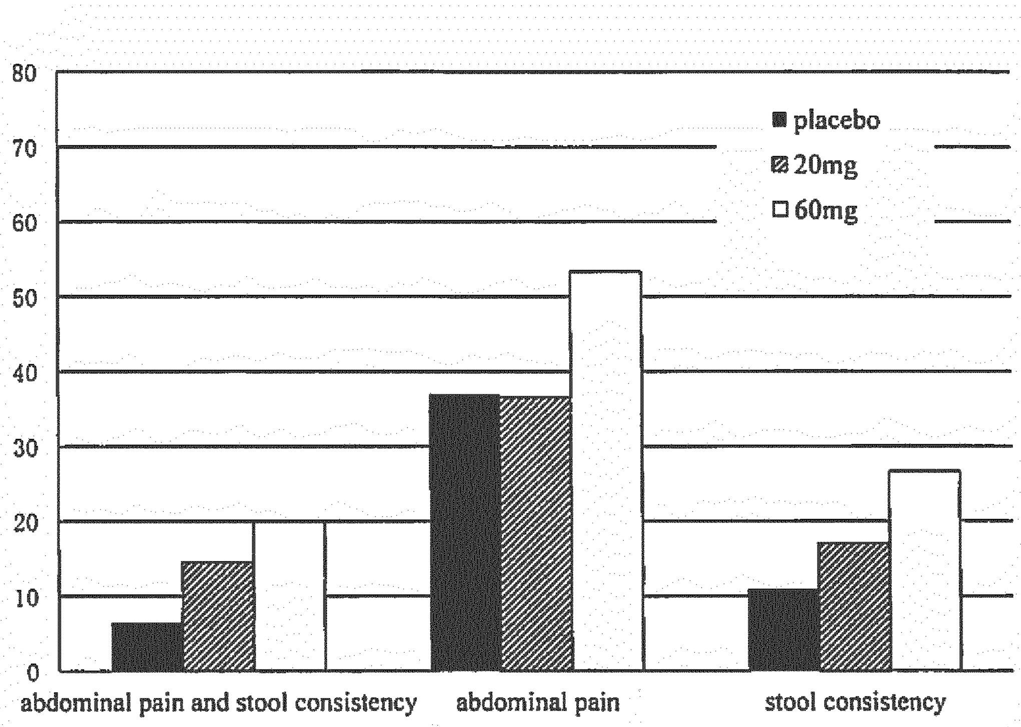
80
70
60
50
40
30
20
10
0
placebo
20mg
60mg
abdominal pain and stool consistency
abdominal pain
stool consistency

Fig.11

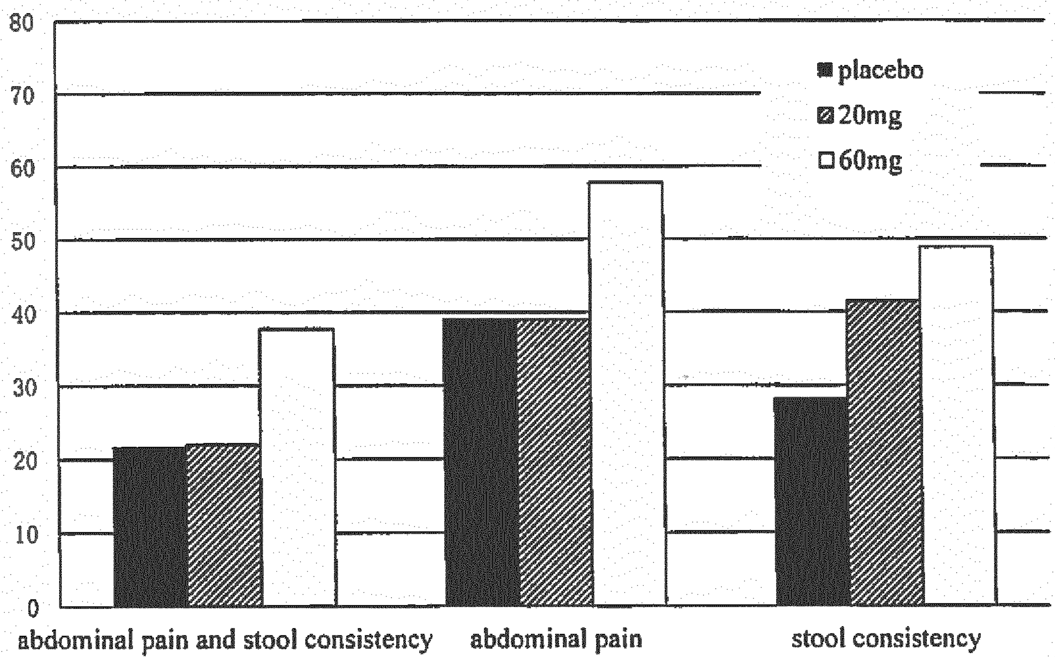
80
70
60
50
40
30
20
10
0
placebo
20mg
60mg
abdominal pain and stool consistency
abdominal pain
stool consistency

**INTERNATIONAL SEARCH REPORT**

International application No.
PCT/JP2015/066922

A. CLASSIFICATION OF SUBJECT MATTER
*A61K31/437*(2006.01)i, *A61K51/00*(2006.01)i, *A61P1/00*(2006.01)i, *A61P1/04*(2006.01)i, *A61P1/12*(2006.01)i, *A61P25/00*(2006.01)i, *A61P25/22*(2006.01)i, *A61P25/24*(2006.01)i, *C07D471/10*(2006.01)n

According to International Patent Classification (IPC) or to both national classification and IPC

B. FIELDS SEARCHED

Minimum documentation searched (classification system followed by classification symbols)
A61K31/437, A61K51/00, A61P1/00, A61P1/04, A61P1/12, A61P25/00, A61P25/22, A61P25/24, C07D471/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2006/068164 A1 (Ono Pharmaceutical Co., Ltd.), 29 June 2006 (29.06.2006), particularly, claims; paragraphs [0004], [0005], [0130], [0144], [0288], [0451] to [0457] & US 2008/0114012 A1 & EP 1829874 A1 | 1-8,10-17<br>1-17 |
| Y | OWEN, D.R.J. et al., Variation in binding affinity of the novel anxiolytic XBD173 for the 18 kDa translocator protein in human brain, Synapse, 2011, vol.65, no.3, p.257-9 | 1-17 |
| Y | Masao IMAIZUMI, "Saishin no Kaku Igaku PET Gijutsu o Kushi shita Shinkei·Men'eki Imaging", Experimental Medicine, 2011, vol.29, no.16, page 2623-8 | 1-17 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 August 2015 (10.08.15) | 25 August 2015 (25.08.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/066922

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions described in claims 1-8 and 10-17 relate to: a prophylactic and/or therapeutic agent for a stress-induced disease, which is characterized in that (1S)-2-acetyl-1-(4-chloro-2-methoxyphenyl)-5-fluoro-1,2,3,9-tetrahydrospiro[β-carboline-4,1'-cyclopropane] (also referred to as "compound A", hereinbelow) is administered in such a unit dose that the occupancy of TSPO in a patient having the stress-induced disease can become about 50% to about 85% or, alternatively, compound A is administered orally at a dose of about 5 mg to about 60 mg once per day; and others.

(Continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**

International application No.
PCT/JP2015/066922

Continuation of Box No.III of continuation of first sheet(2)

On the other hand, the invention described in claim 9 relates to a method for determining the effective dose of a TSPO antagonist for preventing and/or treating a stress-induced disease, said method being characterized by using [$^{11}$C]PBR28 as a radioactive tracer.

In this regard, the relation between TSPO and stress-induced diseases is known (see documents 1 and 2). Therefore, there is not the same or corresponding special technical feature between these inventions.

Accordingly, claims are respectively classified into the following two inventions.

(Invention 1) claims 1-8 and 10-17

·A prophylactic and/or therapeutic agent for a stress-induced disease, which is characterized in that compound A is administered in such a unit dose that the occupancy of TSPO in a patient having the stress-induced disease can become about 50% to about 85%.

·A prophylactic and/or therapeutic agent for irritable bowel syndrome, which is characterized in that compound A is administered orally at a dose of about 5 mg to about 60 mg once per day.

·A method for preventing and/or treating a stress-induced disease, which comprises administering compound A to a patient having the stress-induced disease in such a unit dose that the occupancy of TSPO in the patient having the stress-induced disease can become about 50% to about 85%.

·Compound A which is used for preventing and/or treating a stress-induced disease in such a manner that compound A is administered in such a unit dose that the occupancy of TSPO in a patient having the stress-induced disease can become about 50% to about 85%.

·A prophylactic and/or therapeutic agent for a stress-induced disease, said agent being characterized in that compound A is administered in such a unit dose that the occupancy of TSPO in a patient having the stress-induced disease can become about 50% to about 95%.

(Invention 2) claim 9

·A method for determining the effective dose of a TSPO antagonist for preventing and/or treating a stress-induced disease, said method being characterized by using [$^{11}$C]PBR28 as a radioactive tracer.

Document 1: WO 2006/068164 A1 (Ono Pharmaceutical Co., Ltd.), 29 June 2006 (29.06.2006), & US 2008/0114012 A1 & EP 1829874 A1

Document 2: OWEN, D.R.J. et al., Variation in binding affinity of the novel anxiolytic XBD173 for the 18 kDa translocator protein in human brain, Synapse, 2011, vol.65, no.3, p.257-9

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010049819 A
- WO 2006068164 A


### Non-patent literature cited in the description


- *Proceedings of the National Academy of Science of the United States of America,* 1977, vol. 89, 3805-3809
- *Journal of Clinical Pharmacology,* 2011, vol. 31 (4), 497-502
- *NeuroImage,* 2013, vol. 68, 1-10
- *Proceedings of the National Academy of Science of the United States of America,* 2007, vol. 104 (23), 9800-9805
- *Biological psychiatry,* 2004, vol. 55, 1007-1012
- *The Journal of the American Society for Experimental NeuroTherapeutics,* 2005, vol. 2 (2), 226-236
- *European Journal of Nuclear Medicine and Molecular Imaging,* 22 February 2013
- *The Journal of Nuclear Medicine,* 2011, vol. 52 (5), 677-680
- *CHEMICAL ABSTRACTS,* 85532-75-8
- *CHEMICAL ABSTRACTS,* 253307-65-2
- *CHEMICAL ABSTRACTS,* 386297-97-8
- *Journal of Pharmacological Science,* 2007, vol. 104, 263-273
- **YAMAGUCHI et al.** *Journal of the Pharmaceutical Society of Japan,* 2007, vol. 130, 175-183
- **GOMITA et al.** *Journal of the Pharmaceutical Society of Japan,* 1983, vol. 82, 267-292
- **FUNATSU et al.** *European Journal of Pharmacology,* 2007, vol. 573, 190-195
- **RUPPRECHT et al.** *Science,* 2009, vol. 325, 490-493
- **ROME III.** *Gastroenterology,* 2006, vol. 130, 1480-1491
- *Journal of Health and Social Behavior,* 1983, vol. 24, 386-396
- *The British journal of medical psychology,* 1959, vol. 32, 50-55
- *Journal of Neurology, Neurosurgery and Psychiatry,* 1960, vol. 23, 56-62